(19) Europäisches Patentamt
European Patent Office
Office européen des brevets

(11) **EP 3 803 281 B1**

(12) **EUROPÄISCHE PATENTSCHRIFT**

(45) Veröffentlichungstag und Bekanntmachung des Hinweises auf die Patenterteilung:
**20.11.2024 Patentblatt 2024/47**

(21) Anmeldenummer: **19728383.1**

(22) Anmeldetag: **29.05.2019**

(51) Internationale Patentklassifikation (IPC):
**G01D 21/00** (2006.01)   **A61B 5/11** (2006.01)
**G09B 19/00** (2006.01)   A61B 5/00 (2006.01)
**A61B 5/117** (2016.01)

(52) Gemeinsame Patentklassifikation (CPC):
**G01D 21/00; A61B 5/1118; A61B 5/1122;**
**G09B 19/0038;** A61B 5/1124; A61B 5/117;
A61B 5/7225; A61B 5/7267

(86) Internationale Anmeldenummer:
**PCT/EP2019/064038**

(87) Internationale Veröffentlichungsnummer:
**WO 2019/233868 (12.12.2019 Gazette 2019/50)**

(54) **VORRICHTUNG FÜR KÖRPERÜBUNGEN MIT NÄHERUNGSSENSOR**

DEVICE FOR PHYSICAL EXERCISE  WITH PROXIMITY SENSOR

DISPOSITIF D'EXERCICE PHYSIQUE AVEC CAPTEUR DE PROXIMITÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.06.2018 DE 102018113602**

(43) Veröffentlichungstag der Anmeldung:
**14.04.2021 Patentblatt 2021/15**

(73) Patentinhaber: **ichó systems gmbh**
**47057 Duisburg (DE)**

(72) Erfinder:
• **EFTHIMIADIS, Eleftherios**
**47051 Duisburg (DE)**
• **KNAPPMANN, Mario**
**45711 Datteln (DE)**
• **PREUSS, Steffen**
**47057 Duisburg (DE)**

(74) Vertreter: **Cohausz & Florack**
**Patent- & Rechtsanwälte**
**Partnerschaftsgesellschaft mbB**
**Bleichstraße 14**
**40211 Düsseldorf (DE)**

(56) Entgegenhaltungen:
US-A- 5 512 836     US-A1- 2003 105 390
US-A1- 2013 057 503     US-A1- 2014 309 059
US-A1- 2015 133 820     US-A1- 2017 188 864

**Beschreibung**

Die Erfindung betrifft eine Vorrichtung für Körperübungen, mit einem Gehäuse, mit Sensormitteln zum Bestimmen von Sensordaten indikativ für eine Einwirkung eines Benutzers auf die Vorrichtung, mit Ausgabemitteln zur Ausgabe von Ausgabeinformation an den Benutzer und mit einer Steuereinrichtung, wobei die Steuereinrichtung dafür eingerichtet ist, die Ausgabemittel in Abhängigkeit der Sensordaten zu steuern.

Vorrichtungen für Körperübungen werden beispielsweise für spielerische Aktivitäten und Animation verwendet. Die Vorrichtungen können jedoch auch zum gezielten motorischen und kognitiven Training der Benutzer genutzt werden. Insbesondere körperlich beeinträchtigte Menschen, beispielsweise Menschen in Rehabilitationsphasen nach Verletzungen, ältere oder erkrankte Menschen können von spezialisierten Vorrichtungen für Körperübungen stark profitieren.

Neben klassischen Vorrichtungen wie Turngeräten oder Spielgeräten sind auch interaktive Vorrichtungen entwickelt worden, welche beispielsweise automatisch eine Ausgabe an den Benutzer veranlassen, um einen weiteren Anreiz für bestimmte Trainingsabläufe zu liefern sowie Trainingsprogramme für den Benutzer zu gestalten und zu leiten.

In der WO 01/08755 A1 wird eine interaktive Trainingsmatte beschrieben, welche dem Benutzer die durchzuführenden Übungsschritte anzeigt und Daten zur Leistungsanalyse aufnimmt. Die Trainingsmatte weist ein Laminat zur Detektion von Kontakt mit der Matte und von der Position der Matte auf.

Die WO 2017/152917 A1 betrifft ein reaktionsfähiges Übungsgerät mit einer verformbaren Hülle. Mindestens ein Sensor ist vorgesehen, welcher eine Verformung der Hülle detektiert und eine Sensorausgabe an eine Steuerung veranlasst. Die Steuerung ist konfiguriert, eine audio-visuelle Ausgabe in Abhängigkeit von der Sensorausgabe zu steuern.

Die US 2017/0188864 A1 offenbart ein kugelförmiges, tragbares Biosensor Array-Gerät zur Überwachung eines physiologischen Zustands. Genauer gesagt, werden biomedizinische Geräte offenbart, die periodisch physiologische vitale und nicht-vitale Parameter bei mehreren Benutzern unter Verwendung eines Biosensor-Arrays überwachen.

Die US 2013/0057503 A1 offenbart in einer Ausführungsform ein Verfahren, dass das Leiten eines ersten Signals an eine erste Quellenelektrode außerhalb eines Berührungssensors umfasst. Die erste Quellelektrode ist über ein Berührungsobjekt kapazitiv mit dem Berührungssensor gekoppelt. Das Verfahren umfasst ferner das Messen einer gegenseitigen Kapazität zwischen der ersten Quellenelektrode und der ersten Messelektrode. Das Verfahren umfasst ferner die Identifizierung des Berührungsobjekts, das den Berührungssensor an einer erfassten Berührungsposition berührt, zumindest teilweise auf der Grundlage der gemessenen gegenseitigen Kapazität und unter Verwendung einer Steuerung des Berührungssensors.

Die US 5,512,836 A offenbart einen kapazitiven Festkörper-Näherungssensor mit Streifeneffekt, der sich für die Oberflächenmontage und andere platzbeschränkte Anwendungen eignet. Der Sensor besteht aus einem isolierenden Substrat mit einer Vielzahl von darauf ausgebildeten Fühlerelektroden ohne dazwischen liegende Schirmelemente. Die Elektroden sind so beschaffen, dass sie den Abstand eines (leitenden oder nichtleitenden) Zielobjekts durch den zwischen den Elektroden erzeugten kapazitiven Streifeneffekt messen. Die Schaltung zur Verarbeitung der Kapazitätsmessung ist elektrisch mit der Vielzahl der Sensorelektroden verbunden.

Der vorliegenden Erfindung liegt vor dem Hintergrund des Standes der Technik die Aufgabe zugrunde, eine Vorrichtung für Körperübungen mit erweitertem Funktionsumfang anzugeben. Zudem wird zur Lösung dieser Aufgabe ein Verfahren zur Steuerung einer Vorrichtung für Körperübungen vorgeschlagen.

Gemäß einer ersten Lehre der Erfindung wird die oben genannte Aufgabe bezüglich einer Vorrichtung gemäß dem unabhängigen Anspruch 1 gelöst.

Die Vorrichtung gemäß der ersten Lehre ist für Körperübungen geeignet, wobei die Vorrichtung insbesondere zum motorischen Training als auch zur kognitiven Training der Benutzer genutzt werden kann. Insbesondere ist die Vorrichtung so gestaltet, dass diese als Teil eines Trainings verwendet wird, beispielsweise durch ein Annähern, Berühren, Bewegen und/oder Ansprechen der Vorrichtung durch den Benutzer.

Die Vorrichtung weist ein Gehäuse auf, welches insbesondere die Sensormittel, Ausgabemittel und/oder die Steuereinrichtung umfasst bzw. umschließt. Das Gehäuse weist hierbei eine Form auf, welches eine (trainingsbezogene) Handhabung der Vorrichtung insbesondere durch eine oder mehrere Personen ermöglicht. Die Größe des Gehäuses ist beispielsweise so gewählt, das das Gehäuse mit beiden Händen gehalten und teilweise umschlossen werden kann. Das Gehäuse kann teilweise oder vollständig eine abgerundete Außenkontur aufweisen. Insbesondere weist das Gehäuse eine Abmessung (d.h. eine maximale Abmessung in einer Dimension bzw. eine mittlere Abmessung wie ein Durchmesser) bis zu 20 cm, insbesondere von 8 cm bis 15 cm oder von 11 cm bis 12 cm auf.

Das Gehäuse kann ballförmig sein. Unter "ballförmig" wird hierbei nicht zwangsläufig eine Kugelform verstanden, sondern vielmehr werden hierunter auch Formen verstanden, welche die (trainingsbezogene) Handhabung der Vorrichtung insbesondere durch eine oder mehrere Personen ermöglichen. Beispielsweise hat ein ballförmiges Gehäuse eine Außenkontur, welche im Wesentlichen einem Ellipsoid entspricht, insbesondere einer Kugel. Andere Ballformen sind ebenso denkbar, etwa ein verlängerter Ellipsoid (z.B. ein Football bzw. Pigskin), eine im Wesentlichen zylindrische Form oder die Form eines Kissens.

Das Gehäuse weist insbesondere eine Struktur für eine haptische Anregung auf. Beispielsweise sind auf der Oberfläche des Gehäuses Teilbereiche mit noppenartigen, rippenartigen Strukturen und/oder Vertiefungen vorgesehen, welche dem Benutzer einen haptischen Eindruck liefern. Beispielsweise ist das Gehäuse ballförmig ausgestaltet und weist an gegenüberliegenden Seiten Vertiefungen auf (an den "Polen" des Balls), wobei die Bereiche um die Vertiefungen mit Noppen versehen sind. Der Bereich entlang des Umfangs des Gehäuses (am "Äquator" des Balls) kann Rillen bzw. Erhöhungen aufweisen, welche insbesondere konzentrisch um eine durch die Pole verlaufende Achse verlaufen. Das Gehäuse basiert insbesondere auf Kunststoff und kann beispielsweise die Sensormittel, Ausgabemittel und/oder die Steuereinrichtung wasserdicht umschließen, um eine einfache Reinigung der Vorrichtung zu ermöglichen.

Ausgabemitteln zur Ausgabe von Ausgabeinformation an den Benutzer sind vorgesehen. Die Steuereinrichtung ist hierbei dafür eingerichtet ist, die Ausgabemittel zu steuern. Beispielsweise wird dem Benutzer ein Programmablauf wie eine bestimmte Abfolge von Kombinationen einer Aktivierung, Deaktivierung und/oder Modulation einzelner Ausgabemittel vermittelt, wobei die Abfolge weiter abhängig von Sensordaten ist. Die Ausgabemittel werden zumindest teilweise in Abhängigkeit der Sensordaten gesteuert, d.h. die Einwirkung des Benutzers hat einen mittelbaren oder unmittelbaren Einfluss auf die Ausgabeinformation. Beispielsweise wird die Abfolge von Kombinationen einer Aktivierung, Deaktivierung und/oder Modulation einzelner Ausgabemittel in Abhängigkeit der Sensordaten verändert, z.B. einzelne Abfolgeschritte werden ausgetauscht, wiederholt, unterlassen und/oder in der Intensität verändert.

Die Sensormittel zum Bestimmen von Sensordaten indikativ für eine Einwirkung eines Benutzers auf die Vorrichtung umfassen mindestens einen Näherungssensor. Über einen Näherungssensor kann das Anwendungsgebiet und die Möglichkeiten der Interaktion des Benutzers mit der Vorrichtung erweitert werden. Ein Näherungssensor bewirkt insbesondere den für den Benutzer besonderen Effekt, dass eine Reaktion der Vorrichtung in Form der von den Ausgabemitteln ausgegebenen Ausgabeinformation bereits ohne eine Berührung der Vorrichtung (beispielsweise durch Annähern mit einer Hand) oder durch eine Kombination einer Berührung und einer Annäherung beeinflusst werden kann (beispielsweise ein Halten der Vorrichtung in einer Hand und ein Annähern mit der anderen Hand). Insbesondere können die Sensordaten des Näherungssensors dafür verwendet werden, einen Programmablauf anzukündigen, zu starten oder zu initiieren. Mit dem Näherungssensor lassen sich daher mit der Vorrichtung Körperübungen durchführen und stimulieren, welche über Körperübungen hinausgehen, welche auf reinem Kontakt zur Vorrichtung basieren. Ebenso können über den Näherungssensor Interaktionen mit der Vorrichtung besser erkannt werden. Beispielsweise kann ein Auffangen und ein Werfen der Vorrichtung anhand der Annäherung bzw. Entfernung der Einwirkung auf einfache Weise voneinander unterschieden werden.

Der Näherungssensor ist dafür eingerichtet, Sensordaten indikativ für eine Entfernung der Einwirkung des Benutzers zur Vorrichtung bereitzustellen. Insbesondere handelt es sich bei dem Näherungssensor nicht nur um einen reinen Näherungsschalter, welcher das Unterschreiten einer gewissen Entfernung detektiert. Der Näherungssensor kann insbesondere Sensordaten bereitstellen, welche indikativ für die Entfernung sind bzw. indikativ für eine quantitative Entfernungsinformation sind. Insbesondere sind Auswertemittel im Näherungssensor und/oder der Steuereinrichtung integriert, welche eine quantitative Entfernungsinformation bereitstellen. Die Ausgabeinformation kann wiederum abhängig von den Sensordaten indikativ für eine quantitative Entfernungsinformation ermittelt werden. Eine Entfernung kann hierbei relativ zum Gehäuse der Vorrichtung definiert sein. Über den Näherungssensor wird eine Entfernung der Einwirkung des Benutzers zur Vorrichtung von bis zu 10 cm, insbesondere von bis zu 5 cm detektiert werden, wobei insbesondere innerhalb dieser Entfernung eine quantitative Entfernungsinformation bereitgestellt werden kann.

Die Steuereinrichtung ist oder umfasst etwa eine Datenverarbeitungsanlage, die softwaremäßig und/oder hardwaremäßig eingerichtet ist, um die Ausgabemittel zumindest teilweise in Abhängigkeit der Sensordaten zu steuern. Beispiele für eine Datenverarbeitungsanlage sind ein Computer, beispielsweise ein Kleincomputer, welche auch in die Vorrichtung integriert werden kann und beispielsweise vom Gehäuse umschlossen ist. Die Steuereinrichtung kann zumindest einen Prozessor und zumindest einen Speicher mit Computerprogrammcode umfassen, wobei der zumindest eine Speicher und der Computerprogrammcode dazu eingerichtet sind, die Vorrichtung zu steuern. Die Steuereinrichtung kann eine Kommunikationsschnittstelle umfassen, beispielsweise eine Netzwerkschnittstelle, welche insbesondere dazu eingerichtet ist, eine Verbindung der Vorrichtung mit anderen Vorrichtungen, insbesondere über ein (drahtloses) Kommunikationssystem, beispielsweise ein Netzwerk, herzustellen und mit diesen zu kommunizieren. Beispiele für ein Kommunikationssystem sind ein lokales Netzwerk (LAN), ein großräumiges Netzwerk (WAN), ein drahtloses Netzwerk (beispielsweise gemäß dem IEEE-802.11-Standard, dem Bluetooth (LE)-Standard und/oder dem NFC-Standard), ein drahtgebundenes Netzwerk, ein Mobilfunknetzwerk, ein Telefonnetzwerk und/oder das Internet.

Die Steuereinrichtung kann für ein maschinelles Lernen eingerichtet sein. Unter einem maschinellen Lernen wird verstanden, dass ein künstliches System aus Beispielen lernt und diese nach Beendigung der Lernphase verallgemeinern kann. Das heißt, es werden nicht einfach die Beispiele auswendig gelernt, sondern es werden Muster und Gesetzmäßigkeiten in den Lerndaten erkannt. Hierzu können unterschiedliche Ansätze verfolgt werden. Beispielsweise kann ein überwachtes Lernen, ein teilüberwachtes Lernen, ein unüberwachtes Lernen, ein bestärktes Lernen und/oder ein aktives Lernen eingesetzt werden, insbesondere in Verbindung mit deep learning-Verfahren. Ein überwachtes Lernen kann beispielsweise mittels eines künstlichen neuronalen Netzes (etwa einem rekurrenten neuronalen Netz) oder mittels einer Support Vector Machine erfolgen. Auch ein unüberwachtes Lernen kann beispielsweise mittels eines künstlichen neuronales Netzes (beispielsweis eines Autoencoders) erfolgen. Als Lerndaten dienen beispielsweise die Sensordaten und insbesondere Sensordaten indikativ für eine Entfernung der Einwirkung des Benutzers zur Vorrichtung. Über ein maschinelles Lernen kann beispielsweise die Vorrichtung ein Erkennung von Interaktionen wie bestimmten Gesten und Bewegungsabläufen verbessern.

Verschiedene Funktionsweisen sind für den Näherungssensor denkbar. Beispielsweise können optische Mittel vorgesehen sein (beispielsweise eine Tiefenmessung bzw. Triangulationsmessung, etwa auf Grundlage von Lasern), Ultraschall und/oder Sonar. In einer Ausgestaltung der Vorrichtung gemäß der ersten Lehre ist der mindestens eine Näherungssensor jedoch als kapazitativer Näherungssensor ausgestaltet. Beispielsweise wird mit den Sensormittel eine Kapazitätsmessung mindestens eines Kondensators vorgenommen, wobei der mindestens eine Kondensator im oder am Gehäuse angeordnet ist. Nähert der Benutzer sich der Vorrichtung beispielsweise mit der Hand, kann hierdurch eine Veränderung der Kapazität der Kondensatoren bewirkt und detektiert werden. Hiermit sind erfindungsgemäß auch Sensordaten indikativ für eine quantitative Entfernungsinformation zu ermitteln, beispielsweise indem eine Kapazitätsänderung quantitativ ermittelt wird.

In einer weiteren Ausgestaltung der Vorrichtung gemäß der ersten Lehre wird der kapazitative Näherungssensor über einen Schwingkreis angeregt. Mittels der Anregung über einen Schwingkreis kann über eine Messung der Eigenfrequenz mit hoher Genauigkeit die Kapazität des mindestens einen Kondensators bestimmt werden. Die Frequenzmessung ermöglicht hierbei ebenfalls die Bereitstellung einer quantitativen Entfernungsinformation. Der Schwingkreis wird beispielsweise über eine Oszillatorschaltung bewirkt, insbesondere eine RC-Oszillatorschaltung, LC-Oszillatorschaltung und/oder Ringoszillatorschaltung. Eine RC-Oszillatorschaltung kann beispielsweise unter Verwendung von Schmitt-Triggern aufgebaut werden, womit eine einfache, genaue und kostengünstige Schaltung erhalten wird.

In einer weiteren Ausgestaltung der Vorrichtung gemäß der ersten Lehre weist der kapazitative Näherungssensor Kondensatorelektroden auf, welche halbschalenförmig am Gehäuse angeordnet sind. Durch eine halbschalenförmige Anordnung kann der Näherungssensor im Wesentlichen empfindlich für Einwirkungen des Benutzers in der gesamten Umgebung des Gehäuses sein, wobei ein besonders einfacher Aufbau der Kondensatorelektroden erreicht wird. Die Halbschalen können zudem zur mechanischen Stabilität des Gehäuses beitragen. Prinzipiell erlaubt die Ausbildung als Halbschalen auch eine Bestimmung einer Richtungsabhängigkeit der Einwirkung, da die Kondensatorelektroden verschiedene Polaritäten aufweisen und somit anhand des Vorzeichens bzw. Polarität der Kapazitätsänderung ermittelt werden kann, zu welcher Kondensatorelektrode die Einwirkung näher kommt. Sind etwa Halbschalen oben und unten am Gehäuse vorgesehen, kann bestimmt werden, ob sich die Hand eines Benutzers von oben oder unten nähert. Die Sensordaten können somit auch indikativ für eine Information bezüglich der Richtung der Einwirkung sein, d.h. der Näherungssensor kann dafür eingerichtet sein, Sensordaten indikativ für eine Richtungsabhängigkeit der Einwirkung des Benutzers bereitzustellen.

Die Halbschalen können entsprechend der Form des Gehäuses ausgebildet sein. Für das kugelförmige Gehäuse sind beispielsweise Kugelabschnitte bzw. Kugelkalotten vorgesehen, wobei die Halbschalen z.B. an der Innenfläche der Außenwand des Gehäuses angeordnet sind. Für die Halbschalen bzw. Kondensatorelektroden sind verschiedene Materialien denkbar, etwa Stahl, Kupfer, Gold, Silber und/oder Zinn, wobei vorzugsweise kupferhaltige Materialien wie Kupfer oder Kupferlegierungen verwendet werden.

Der kapazitative Näherungssensor weist mehrere Kondensatorbereiche mit jeweils mindestens zwei Kondensatorelektroden auf. Entsprechend können die Kapazitäten mehrerer Kondensatoren bestimmt und miteinander abgeglichen werden. Hierdurch kann einerseits die quantitative Entfernungsinformation genauer bestimmt werden. Neben einer genaueren Ermittlung der Entfernung (d.h. dem Maß von der Vorrichtung bzw. dem Gehäuse zum Benutzer, etwa der Hand des Benutzers) können die Sensordaten andererseits auch indikativ für eine Richtungsabhängigkeit der Einwirkung des Benutzers sein. In einer einfachen Ausgestaltung sind zwei Elektroden als Halbschalen ausgebildet und zwei weitere Elektroden im Inneren des Gehäuses angeordnet, so dass die Seite der Vorrichtung, an welcher die Einwirkung stattfindet, auf einfache Weise bestimmt werden kann, beispielsweise über den Vergleich der Kapazitätsmessungen verschiedener Kondensatorbereiche.

In einer weiteren Ausgestaltung der Vorrichtung gemäß der ersten Lehre umfassen die Sensormittel weiter mindestens einen Drucksensor, mindestens ein Beschleunigungssensor, mindestens ein Gyroskop, mindestens einen Kompass, mindestens einen Geschwindigkeitssensor, mindestens ein Positionsbestimmungsmittel und/oder mindestens ein akustisches Sensormittel. Ein Drucksensor kann beispielsweise ein barometrischer Sensor sein, welcher eine Druckveränderung durch eine Verformung des Gehäuses bei Berührung detektieren kann. Ebenso kann ein Drucksensor einen Schalter wie einen elektronischen und/oder mechanischen Schalter umfassen, welcher am Gehäuse angeordnet ist und beispielsweise über eine Verformung des Gehäuses betätigt werden kann. Prinzipiell können Drucksensoren auch an der Außenseite des Gehäuses angeordnet sein. Eine

Kombination von Gyroskop und Kompass können der Bestimmung der räumlichen Lage der Vorrichtung dienen. Als akustisches Sensormittel ist beispielsweise mindestens ein Mikrofon vorgesehen. Weitere denkbare Sensormittel sind mindestens ein Geschwindigkeitssensor und/oder ein Positionsbestimmungsmittel (z.B. ein GPS).

In einer weiteren Ausgestaltung der Vorrichtung gemäß der ersten Lehre ist das Gehäuse austauschbar ausgestaltet. Das Gehäuse bildet beispielsweise eine Hülle, welche geöffnet und durch eine andere Hülle ersetzt werden kann. Beispielsweise weist das Gehäuse zwei oder mehr Teile auf, welche über Verbindungsmittel miteinander verbunden und wieder voneinander gelöst werden können. Beispielsweise sind zwei Hälften eines Gehäuses über ein Gewinde miteinander verbunden. Die weiteren Bauteile der Vorrichtung können vom Gehäuse wasserdicht umschlossen werden. Das Gehäuse kann beispielsweise ausgetauscht werden, um eine Struktur für eine haptische Anregung anzupassen.

Ebenso können weitere Elemente der Vorrichtung in dem Gehäuse integriert sein und mit dem Austauschen des Gehäuses ebenfalls ausgewechselt werden. Beispielsweise ist zumindest ein Teil der Sensormittel in dem austauschbaren Gehäuse integriert. Wie zuvor beschrieben, sind beispielsweise Drucksensoren vorgesehen, welche einen Kontakt des Benutzers mit dem Gehäuse detektieren können. Beispielsweise sind Drucksensoren im Gehäuse integriert, so dass durch ein Austauschen des Gehäuses die Position und/oder Anzahl der Drucksensoren verändert werden kann. Ebenso denkbar ist ein Integrieren zumindest eines Teils des Näherungssensors im Gehäuse, beispielsweise ein Integrieren der Kondensatorelektroden. Entsprechend kann mit einem Austauschen des Gehäuses beispielsweise die Sensitivität des Näherungssensors angepasst werden.

In einer weiteren Ausgestaltung der Vorrichtung gemäß der ersten Lehre sind die Ausgabemittel zur Ausgabe von optischer, mechanischer und/oder akustischer Ausgabeinformation eingerichtet. Optische Ausgabemittel können beispielsweise Leuchtmittel und insbesondere LEDs umfassen. Insbesondere sind weiße LEDs und/oder RGB-LEDs zur Farbmischung vorgesehen, wobei verschiedene Farben und Helligkeiten ausgegeben werden können. Beispielsweise ist eine LED-Blende vorgesehen, d.h. eine Anordnung von LEDs an einem umlaufenden Bereich des Gehäuses. Akustische Ausgabemittel umfassen beispielsweise einen oder mehrere Lautsprecher.

Als mechanische Ausgabemittel ist beispielsweise ein Vibrationselement vorgesehen. Insbesondere wird ein Vibrationsmotor verwendet, welcher einen Rotationskörper mit einer Unwucht drehen kann. Mechanische Ausgabemittel sind insbesondere mit einer Struktur des Gehäuses für eine haptische Anregung vorteilhaft, beispielsweise mit Teilbereichen mit noppenartigen, rippenartigen Strukturen und/oder Vertiefungen auf der Oberfläche des Gehäuses, da die Haptik durch die mechanische Ausgabeinformation verstärkt wird.

Ein Nutzerprofil wird erhalten, beispielsweise von einer Steuereinrichtung der Vorrichtung. Das Nutzerprofil ist einem Benutzer der Vorrichtung zugeordnet, beispielsweise wird ein Nutzerprofil anhand der Identität des Benutzers ausgewählt oder bestimmt. Beispielsweise kann ein Benutzer ein Nutzerprofil über eine Kommunikationsschnittstelle aus einer Liste von hinterlegten Nutzerprofilen auswählen, insbesondere über eine optische Anzeige- und Eingabemittel und/oder eine akustische Eingabe, wobei die Anzeige- und Eingabemittel nicht Teil der Vorrichtung für Körperübungen sein müssen. Beispielsweise wird das Nutzerprofil anhand charakteristischer Merkmale des Benutzers bestimmt, beispielsweise anhand des typischen Aufenthaltsorts des Benutzers und der (globalen) Position der Vorrichtung.

Eine Ausgabefunktion wird aus einer Mehrzahl von vorbestimmten Vorgabefunktionen anhand des Nutzerprofils bestimmt, insbesondere durch die Steuereinrichtung. Vorbestimmte Vorgabefunktionen können beispielsweise hinterlegte Trainingsprogramme bzw. Trainingsabläufe sein. Eine Vorgabefunktion kann beispielsweise einen Programmablauf wie eine bestimmte Abfolge von Kombinationen einer Aktivierung, Deaktivierung und/oder Modulation einzelner Ausgabemittel umfassen. Die Abfolge von Kombinationen einer Aktivierung, Deaktivierung und/oder Modulation einzelner Ausgabemittel ist zumindest teilweise abhängig von Sensordaten indikativ für eine Einwirkung eines Benutzers auf die Vorrichtung, wobei die Abhängigkeit von den Sensordaten ebenfalls durch die Vorgabefunktion bestimmt sein kann.

Die Bestimmung der Ausgabefunktion umfasst insbesondere ein Auswählen einer Vorgabefunktion anhand des Nutzerprofils. Beispielsweise wird für den Benutzer ein bestimmtes Trainingsprogramm gemäß einer Empfehlung ausgewählt, beispielsweise das gleiche Trainingsprogramm für jeden Tag (etwa bei einem bestimmten Krankheitsbild) oder wechselnde Trainingsprogramme (beispielsweise nach einer Vorgabe eines langfristigen Trainingsablaufs abhängig vom aktuellen Datum). Ebenso können einzelne für das Trainingsprogramm charakteristische Merkmale über das Nutzerprofil bestimmt werden. Beispiele solcher charakteristischer Merkmale sind Intensität, Dauer, Geschwindigkeit einer Abfolge, das Aktivieren und/oder Deaktivieren einzelner Ausgabemittel.

Durch die Verwendung von Nutzerprofilen kann eine Personalisierung des Trainingsprogramms der Vorrichtung für Körperübungen durchgeführt werden. Das Nutzerprofil erlaubt eine Optimierung des Trainingsprogramms einzelner Benutzer, wobei insbesondere das Nutzerprofil auch von weiteren Personen, beispielsweise einem Trainer oder Betreuer, auf einfache Weise überwacht und angepasst werden kann. Zudem können eine Mehrzahl von Nutzerprofilen für mehrere Benutzer angelegt werden, so dass eine Vorrichtung für Körperübungen von mehreren Benutzern verwendet werden kann.

Sensordaten indikativ für eine Einwirkung eines Benutzers auf die Vorrichtung werden erhalten, insbesondere werden Sensordaten von Sensormitteln bereitgestellt und von einer Steuereinrichtung erhalten. Ausgabeinformation wird zumindest anhand der Sensordaten und der Ausgabefunktion bestimmt, insbesondere von der Steuereinrichtung. Ein Ausgeben der Ausgabeinformation kann von Ausgabemitteln durchgeführt werden und wird insbesondere von der Steuereinrichtung veranlasst bzw. gesteuert.

Gemäß der zweiten Lehre der Erfindung wird auch eine Vorrichtung beschrieben, umfassend zumindest einen Prozessor und zumindest einen Speicher mit Computerprogrammcode, wobei der zumindest eine Speicher und der Computerprogrammcode dazu eingerichtet sind, mit dem zumindest einen Prozessor zumindest ein Verfahren gemäß der zweiten Lehre auszuführen und/oder zu steuern. Unter einem Prozessor soll zum Beispiel eine Kontrolleinheit, ein Mikroprozessor, eine Mikrokontrolleinheit wie ein Mikrocontroller, ein digitaler Signalprozessor (DSP), eine anwendungsspezifische Integrierte Schaltung (ASIC) oder ein Field Programmable Gate Arrays (FPGA) verstanden werden.

Zum Beispiel umfasst eine beispielhafte Vorrichtung ferner Mittel zum Speichern von Informationen wie einen Programmspeicher und/oder einen Hauptspeicher. Zum Beispiel umfasst eine beispielhafte erfindungsgemäße Vorrichtung ferner jeweils Mittel zum Empfangen und/oder Senden von Informationen über ein Netzwerk wie eine Netzwerkschnittstelle. Zum Beispiel sind beispielhafte erfindungsgemäße Vorrichtungen über ein oder mehrere Netzwerke miteinander verbunden und/oder verbindbar.

Eine beispielhafte Vorrichtung gemäß der zweiten Lehre ist oder umfasst etwa eine Datenverarbeitungsanlage, die softwaremäßig und/oder hardwaremäßig eingerichtet ist, um die jeweiligen Schritte eines beispielhaften Verfahrens gemäß der zweiten Lehre ausführen zu können. Beispiele für eine Datenverarbeitungsanlage sind ein (Klein-)Computer, ein Desktop-Computer, ein Server, ein Thinclient und/oder ein tragbarer Computer (Mobilgerät), wie etwa ein Laptop-Computer, ein Tablet-Computer, ein Wearable, ein persönlicher digitaler Assistent oder ein Smartphone.

Offenbart wird zudem die Verwendung eines Näherungssensors, welcher dafür eingerichtet ist, Sensordaten indikativ für eine Entfernung der Einwirkung des Benutzers zu einer Vorrichtung bereitzustellen, in einer Vorrichtung für Körperübungen, mit einem Gehäuse, mit Sensormitteln zum Bestimmen von Sensordaten indikativ für eine Einwirkung eines Benutzers auf die Vorrichtung, mit Ausgabemitteln zur Ausgabe von Ausgabeinformation an den Benutzer und mit einer Steuereinrichtung, wobei die Steuereinrichtung dafür eingerichtet ist, die Ausgabemittel in Abhängigkeit der Sensordaten zu steuern.

Die zuvor in dieser Beschreibung beschriebenen beispielhaften Ausgestaltungen der vorliegenden Erfindung sollen auch in allen Kombinationen miteinander offenbart verstanden werden. Insbesondere sollen beispielhafte Ausgestaltungen in Bezug auf die unterschiedlichen Lehren offenbart verstanden werden.

Weitere Ausgestaltungen und Vorteile der Erfindung sind in der folgenden detaillierten Beschreibung einiger beispielhafter Ausführungsformen der vorliegenden Erfindung, insbesondere in Verbindung mit der Zeichnung, zu entnehmen. Die Zeichnung zeigt in

Fig. 1 ein Ausführungsbeispiel einer Vorrichtung gemäß der ersten Lehre als schematische Darstellung in einer Außenansicht,

Fig. 2 die Vorrichtung aus Fig. 1 als schematische Darstellung in einer Explosionsansicht,

Fig. 3 ein weiteres Ausführungsbeispiel einer Vorrichtung gemäß der ersten Lehre in einer schematischen Ansicht,

Fig. 4 eine schematische Darstellung einer Steuereinrichtung für eine Vorrichtung gemäß der ersten Lehre,

Fig. 5 eine schematische Darstellung eines Schaltkreises für einen Näherungssensor,

Fig. 6 ein schematisches Ablaufdiagramm eines Ausführungsbeispiels eines Verfahrens, das nicht Teil der Erfindung ist,

Fig. 7 ein Blockdiagramm eines Ausführungsbeispiels einer Vorrichtung und

Fig. 8 unterschiedliche Ausführungsbeispiele eines Speichermediums.

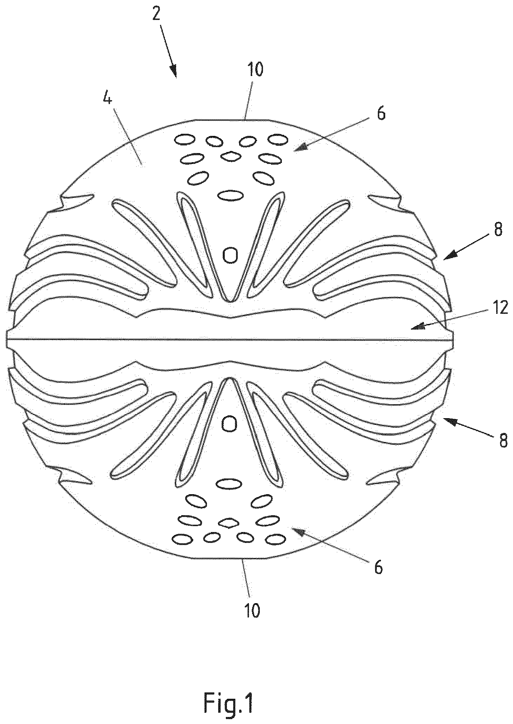

Fig. 1 zeigt ein Ausführungsbeispiel einer Vorrichtung 2 gemäß der ersten Lehre als schematische Darstellung in einer Außenansicht, wobei die Vorrichtung 2 ein Gehäuse 4 aufweist. Das Gehäuse 4 ist hierbei ballförmig und weist im Wesentlichen die Form einer Kugel auf. Das Gehäuse 4 weist eine Struktur für eine haptische Anregung auf. Auf der Oberfläche des Gehäuses 4 sind Teilbereiche mit noppenartigen Strukturen 6, rippenartigen Strukturen 8 und Vertiefungen 10 vorgesehen, welche einen haptischen Effekt vermitteln. Das Gehäuse 4 basiert auf Kunststoff. Eine LED-Blende 12 mit einer Anordnung von LEDs an einem umlaufenden Bereich des Gehäuses 4 ist vorgesehen.

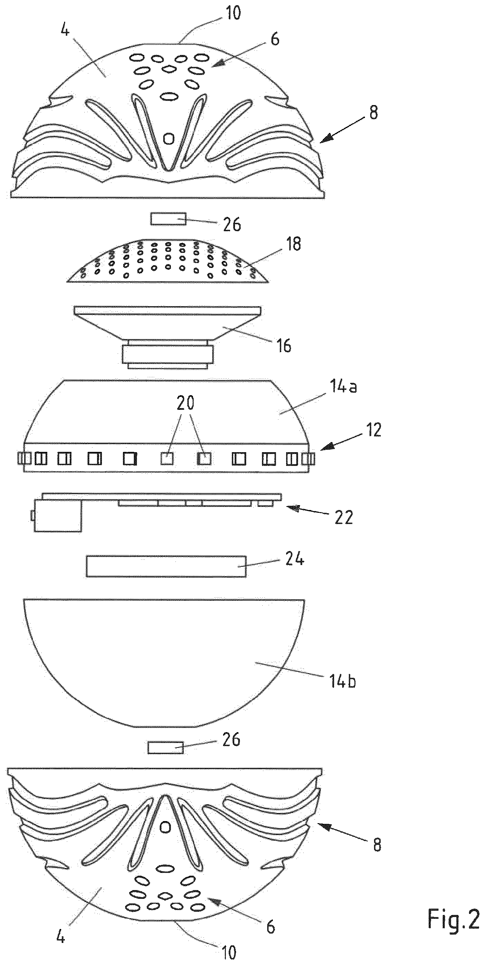

Fig. 2 zeigt die Vorrichtung 2 aus Fig. 1 als schematische Darstellung in einer Explosionsansicht. Das Gehäuse 4 weist hierbei zwei Hälften auf, welche beispielsweise über ein Gewinde miteinander verbunden werden können. Die weiteren Bauteile der Vorrichtung 2 können hiermit wasserdicht umschlossen werden, um eine einfache Reinigung der Vorrichtung 2 zu ermöglichen. Über das Gewinde können die Hälften des Gehäuses voneinander gelöst werden, um Zugriff auf die weiteren Bauteile im Inneren der Vorrichtung 2 zu erhalten. Weiter ist das Gehäuse 4 austauschbar gestaltet, so dass beispielsweise ein Gehäuse mit einer anderen Struktur verwendet werden kann. Zudem können zumindest Teile der Sensormittel im Gehäuse 4 integriert und austauschbar sein.

Sensormittel zum Bestimmen von Sensordaten indikativ für eine Einwirkung eines Benutzers auf die Vorrichtung 2 sind vorgesehen. Die Sensormittel umfassen einen Näherungssensor, welcher dafür eingerichtet ist, Sensordaten indikativ für eine Entfernung der Einwirkung des Benutzers zur Vorrichtung 2 bereitzustellen. Der Näherungssensor ist als kapazitativer Näherungssensor ausgestaltet und weist Kondensatorelektroden 14a, 14b auf, welche halbschalenförmig am Gehäuse 4 angeordnet sind. Die Kondensatorelektroden 14a, 14b sind beispielsweise als Kupferhalbschalen, Kupferfolie oder Kupfergewebe ausgebildet.

Ausgabemitteln zur Ausgabe von optischer, mechanischer und akustischer Ausgabeinformation an den Benutzer sind vorgesehen. Zur akustischen Ausgabe ist ein Lautsprecher 16 mit einer Abdeckung 18 vorgesehen. Optische Ausgabemittel sind in der LED-Blende 12 in Form von RGB-LED-Elementen 20 vorgesehen, welche umlaufend entlang des Umfangs des Gehäuses 4 angeordnet sind. Eine Platine 22 für eine Steuereinrichtung ist vorgesehen, welche dafür eingerichtet ist, die Ausgabemittel in Abhängigkeit der Sensordaten zu steuern. Die Vorrichtung 2 wird über eine Batterie 24 mit elektrischer Energie versorgt. Als weitere Sensormittel sind Drucksensoren 26 vorgesehen, welche im Bereich der Vertiefungen 10 angeordnet sind, so dass ein Aktivieren der Drucksensoren 26 durch Ausübung von Druck bzw. Berührung des Gehäuses 4 an den Vertiefungen 10 ermöglicht wird.

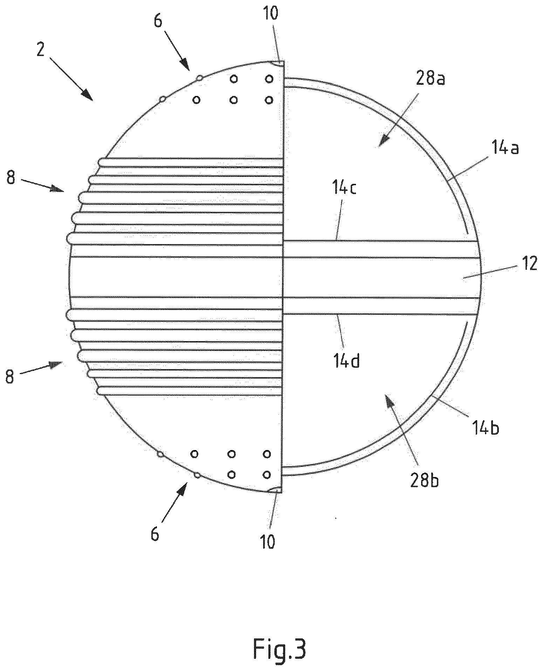

Fig. 3 zeigt ein weiteres Ausführungsbeispiel einer Vorrichtung 2 gemäß der ersten Lehre in einer schematischen Ansicht, wobei entsprechende Elemente mit den gleichen Bezugszeichen gezeigt sind wie in den Fig. 1 und 2. Das Gehäuse 4 weist eine andere Struktur für eine haptische Anregung mit noppenartigen Strukturen 6, umlaufenden rippenartigen Strukturen 8 und Vertiefungen 10 auf.

Der Näherungssensor ist ebenfalls als kapazitativer Näherungssensor ausgestaltet und weist Kondensatorelektroden 14a, 14b auf, welche halbschalenförmig am Gehäuse 4 angeordnet sind. In Fig. 3 ist jedoch ein kapazitativer Näherungssensor mit mehreren Kondensatorbereichen 28a, 28b und jeweils zwei Kondensatorelektroden 14a, 14c; 14b, 14d gezeigt. Die Kondensatorelektroden 14a, 14b weisen hierbei die Form eines Kugelabschnitts bzw. einer Kugelkalotte auf, während die Kondensatorelektroden 14c, 14d kreisförmig bzw. plattenförmig ausgestaltet und benachbart zur LED-Blende 12 angeordnet sind. Der Näherungssensor kann somit auf einfache Weise Sensordaten indikativ für eine Richtungsabhängigkeit der Einwirkung des Benutzers bereitstellen, beispielsweise indem die Kapazitäten beider Kondensatorbereiche 28a, 28b bzw. der Kondensatorelektroden 14a, 14c und der Kondensatorelektroden 14b, 14d jeweils ausgewertet werden.

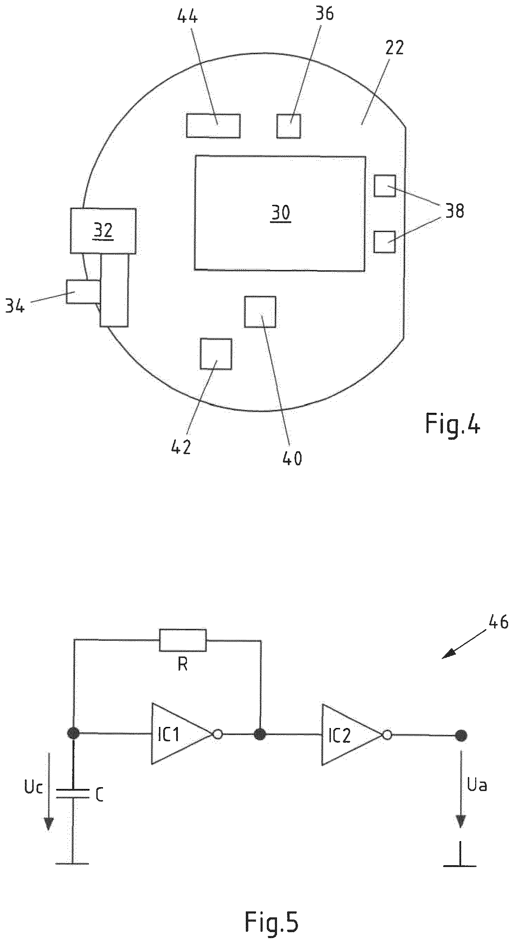

Fig. 4 zeigt eine schematische Darstellung einer Steuereinrichtung 30 für eine Vorrichtung 2, wobei die Steuereinrichtung 30 auf einer Platine 22 angeordnet ist. Die Steuereinrichtung 30 ist als Kleincomputer mit einem Prozessor, einem Speicher mit Computerprogrammcode ausgestaltet, wobei der Speicher und der Computerprogrammcode dazu eingerichtet sind, die Vorrichtung 2 zu steuern. Die Steuereinrichtung 30 weist zudem eine Kommunikationsschnittstelle auf (z.B. zur Kommunikation über Bluetooth oder WLAN). Eine Ladebuchse 32 zum Anschluss an eine Stromversorgung ist vorgesehen, wobei alternativ oder kumulativ auch Mittel zum kabellosen Aufladen vorgesehen sein können, z.B. Mittel zur induktiven Aufladung. Ein Bedienelement 34 kann beispielsweise als Schalter zum Ein- und Ausschalten der Vorrichtung 2 dienen.

Auf der Platine 22 ist weiter ein Drucksensor 36 in Form eines Barometers angeordnet, welcher beispielsweise eine Druckveränderung aufgrund einer Verformung des Gehäuses 4 mittels einer Berührung ermitteln kann. Als akustische Sensormittel sind Mikrofone 38 vorgesehen. Eine Kombination aus Beschleunigungssensor, Gyroskop und Kompass 40 ist ebenfalls auf der Platine 22 angeordnet. Als mechanisches Ausgabemittel ist ein Vibrationselement 42 vorgesehen.

Der kapazitative Näherungssensor kann über einen Schwingkreis angeregt werden, wobei die Eigenfrequenz des Schwingkreises ausgewertet wird. Die Auswertemittel für den Schwingkreis können hierbei in die Sensormittel integriert sein oder Auswertemittel 44 sind ebenfalls auf der Platine 22 angeordnet.

Fig. 5 zeigt eine schematische Darstellung eines Schaltkreises 46 für einen Näherungssensor, wie dieser beispielsweise in den Auswertemitteln 44 realisiert ist. Der Schaltkreis 46 weist Schmitt-Trigger IC1 und IC2 auf, wobei Schmitt-Trigger IC1 parallel zu Widerstand R geschaltet ist. Schmitt-Trigger IC 1 und IC2 können als Bauelemente 74HC14 ausgestaltet sein. Uc bezeichnet hierbei die über dem Kondensatorbereich des Näherungssensors mit Kapazität C abfallende Spannung sowie Ua die Ausgangsspannung. Die Frequenz bestimmt sich hierbei über

$$f = 1/(1{,}45 \text{ x } R \text{ x } C),$$

so dass über eine Bestimmung der Frequenz $f$ die Kapazität C ermittelt werden kann. Die Kapazität C wiederum verändert sich bei Annäherung einer Einwirkung des Benutzers, so dass Sensordaten indikativ für eine quantitative Entfernungsinformation bereitgestellt werden können. Insbesondere kann eine quantitative Entfernungsinformation für Entfernungen von bis zu 5 cm, insbesondere von bis zu 10 cm bereitgestellt werden.

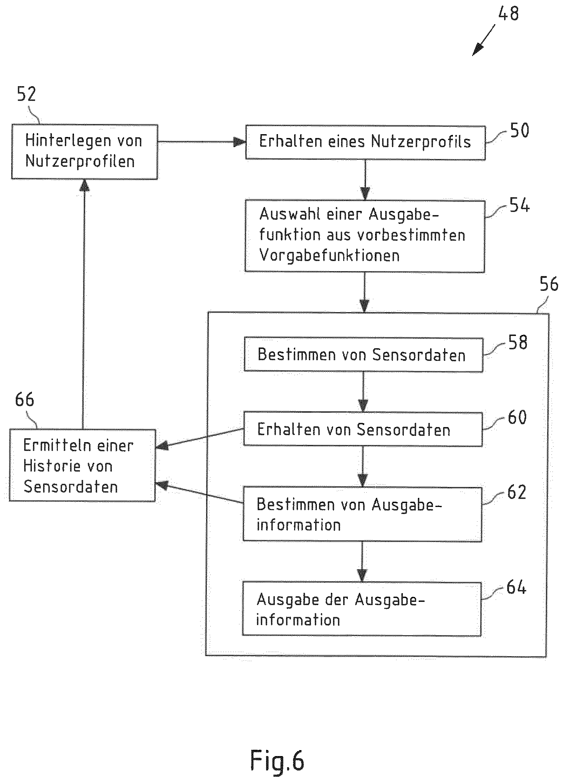

Fig. 6 zeigt ein schematisches Ablaufdiagramm eines Verfahrens, das nicht Teil der Erfindung ist.

Das Verfahren 48 dient zur Steuerung einer Vorrichtung 2 für Körperübungen.

In Aktion 50 wird ein Nutzerprofil, welches einem Benutzer der Vorrichtung 2 zugeordnet ist, erhalten, etwa von einer Steuereinrichtung oder sonstigen Datenverarbeitungsanlage. Beispielsweise ist eine Mehrzahl von Nutzerprofilen für verschiedene Benutzer der gleichen Vorrichtung 2 in einer Datenbank entsprechend Aktion 52 hinterlegt. Beispielsweise wird ein Nutzerprofil von einem Benutzer ausgewählt oder von der Vorrichtung 2 bestimmt.

In Aktion 54 erfolgt eine Auswahl einer Ausgabefunktion aus einer Mehrzahl von vorbestimmten Vorgabefunktionen anhand des erhaltenen Nutzerprofils.

Vorbestimmte Vorgabefunktionen können beispielsweise hinterlegte Trainingsprogramme sein, etwa eine bestimmte Abfolge von Kombinationen einer Aktivierung, Deaktivierung und/oder Modulation einzelner Ausgabemittel und deren Abhängigkeit von Sensordaten. Beispielsweise enthält das Nutzerprofil eine Empfehlung eines Trainingsprogramms für ein bestimmtes Krankheitsbild oder Trainingsziel des Benutzers und/oder persönliche Präferenzen hinsichtlich der Ausgabemittel.

Mit der Auswahl der Ausgabefunktion wird beispielsweise ein Trainingsprogramm 56 initiiert. Die hierbei ausgeführten Aktionen sind insbesondere interaktiv, d.h. beispielsweise werden regelmäßig Sensordaten indikativ für eine Einwirkung eines Benutzers auf die Vorrichtung 2 in Aktion 58 bestimmt und eine Reaktion der Vorrichtung 2 hierauf erzeugt. In Aktion 60 werden die Sensordaten indikativ für eine Einwirkung eines Benutzers auf die Vorrichtung 2 erhalten, beispielsweise durch eine Steuereinrichtung 30. Die Steuereinrichtung bestimmt in Aktion 62 Ausgabeinformation zumindest anhand der Sensordaten und der Ausgabefunktion. In Aktion 64 wird ein Ausgeben der Ausgabeinformation veranlasst, indem die Steuereinrichtung 30 die Ausgabemittel steuert.

Ein Beispiel für ein Trainingsprogramm, welches eine Annäherung an die Vorrichtung 2 sowie die Bewegung der Vorrichtung 2 fördert, beginnt über eine Abhängigkeit der Helligkeit der LEDs 20 der LED-Blende. Abhängig von Sensordaten indikativ für eine Entfernung der Einwirkung des Benutzers zur Vorrichtung 2 werden die LEDs 20 mit Annäherung bzw. reduzierter Entfernung heller. Hierbei kann eine Richtungsabhängigkeit der Sensordaten des Näherungssensors genutzt werden, z.B. indem die LEDs 20 entsprechend der Annäherungsrichtung des Benutzers unterschiedlich hell leuchten oder mit verschiedenen Farben betrieben werden. Berührt der Benutzer die Vorrichtung 2, was beispielsweise über die Drucksensoren 26, 36 ermittelt wird, wird ein akustisches Signal über den Lautsprecher 16 ausgegeben und die LEDs 20 geben grünes Licht aus. Über den Beschleunigungssensor, Gyroskop und Kompass 40 wird ermittelt, ob der Benutzer die Vorrichtung entsprechend einem vorgegebenen Bewegungsmuster in Bewegung hält, wobei bei einem Einhalten des Bewegungsmusters die LEDs 20 weiterhin grün leuchten. Werden Abweichungen vom Bewegungsmuster ermittelt und er Benutzer bewegt beispielsweise die Vorrichtung 2 nicht mehr, kann die Farbe der LEDs 20 mit einer zeitlichen Abhängigkeit von grün nach rot verschoben werden. Mit einer roten Farbe der LEDs 20 kann gleichzeitig ein Vibrationsimpuls über Vibrationselement 42 sowie ein akustisches Signal über den Lautsprecher 16 ausgegeben werden. Die Helligkeit der LEDs 20 kann dann beispielsweise auch wiederum abhängig von der Entfernung der Einwirkung des Benutzers gesteuert werden, etwa falls der Benutzer die Vorrichtung 2 loslässt. Ein Aktivieren und Deaktivieren der Vorrichtung 2 kann beispielsweise über Schalter 34 durchgeführt werden.

Das Nutzerprofil ist indikativ für eine Historie von Sensordaten, welche dem Benutzer zugeordnet sind, wobei die Auswahl der Ausgabefunktion in Aktion 54 abhängig von der Historie von Sensordaten durchgeführt wird. Hierzu wird eine Historie von Sensordaten in Aktion 66 ermittelt, wobei insbesondere neben den Sensordaten auch Ausgabeinformation hinterlegt wird. Beispielsweise kann somit der Verlauf einer Reaktion auf bestimmte Ausgabeinformation ausgewertet werden. Beispielsweise kann die Dauer und/oder Intensität einzelner Trainingsprogramme bestimmt und festgehalten werden. Insbesondere wird eine statistische Auswertung der Historie vorgenommen, um Nutzerprofile entsprechend anzupassen. Insbesondere wird ein maschinelles Lernen verwendet (z.B. über ein künstliches neuronales Netz), um für den jeweiligen Benutzer individuelle Merkmale von Bewegungen und Gesten zu erfassen und zu erkennen. Nutzerprofile können hierbei über das maschinelle Lernen angepasst werden.

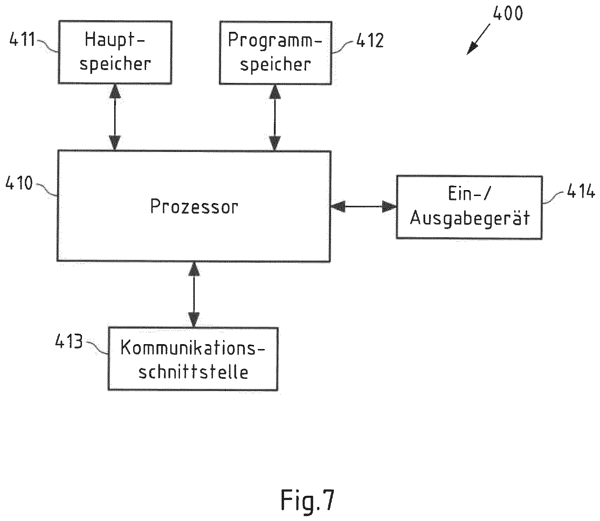

Fig. 7 zeigt ein Blockdiagramm eines Ausführungsbeispiels einer Vorrichtung 400, welche insbesondere ein beispielhaftes Verfahren gemäß der ersten und/oder zweiten Lehre ausführen kann. Die Vorrichtung 400 ist beispielsweise eine Vorrichtung gemäß der zweiten Lehre oder zumindest ein Teil einer Vorrichtung gemäß der ersten Lehre, insbesondere eine Steuereinheit.

Die Vorrichtung 400 kann insofern beispielsweise ein (Klein-)Computer, ein Desktop-Computer, ein Server, ein Thinclient oder ein tragbarer Computer (Mobilgerät), wie etwa ein Laptop-Computer, ein Tablet-Computer, ein persönlicher digitaler Assistent (PDA) oder ein Smartphone sein. Die Vorrichtung kann beispielsweise die Funktion eines Servers oder eines Clients erfüllen.

Prozessor 410 der Vorrichtung 400 ist insbesondere als Mikroprozessor, Mikrokontrolleinheit, Mikrocontroller, digitaler Signalprozessor (DSP), Anwendungsspezifische Integrierte Schaltung (ASIC) oder Field Programmable Gate Array (FPGA) ausgebildet.

Prozessor 410 führt Programmanweisungen aus, die in Programmspeicher 412 gespeichert sind, und speichert beispielsweise Zwischenergebnisse oder ähnliches in Arbeits- oder Hauptspeicher 411. Zum Beispiel ist Programmspeicher 412 ein nicht-flüchtiger Speicher wie ein Flash-Speicher, ein Magnetspeicher, ein EEPROM-Speicher (elektrisch löschbarer programmierbarer Nur-Lese-Speicher) und/oder ein optischer Speicher. Hauptspeicher 411 ist zum Beispiel ein flüchtiger oder nicht-flüchtiger Speicher, insbesondere ein Speicher mit wahlfreiem-Zugriff (RAM) wie ein statischer RAM-Speicher (SRAM), ein dynamischer RAM-Speicher (DRAM), ein ferroelektrischer RAM-Speicher (FeRAM) und/oder ein magnetischer RAM-Speicher (MRAM).

Programmspeicher 412 ist vorzugsweise ein lokaler mit der Vorrichtung 400 fest verbundener Datenträger. Mit der Vorrichtung 400 fest verbundene Datenträger sind beispielsweise Festplatten, die in die Vorrichtung 400 eingebaut sind. Alternativ kann der Datenträger beispielsweise auch ein mit der Vorrichtung 400 trennbar verbindbarer Datenträger sein wie ein Speicher-Stick, ein Wechseldatenträger, eine tragbare Festplatte, eine CD, eine DVD und/oder eine Diskette.

Programmspeicher 412 enthält beispielsweise das Betriebssystem von der Vorrichtung 400, das beim Starten der Vorrichtung 400 zumindest teilweise in Hauptspeicher 411 geladen und vom Prozessor 410 ausgeführt wird. Insbesondere wird beim Starten von Vorrichtung 400 zumindest ein Teil des Kerns des Betriebssystems in den Hauptspeicher 411 geladen und von Prozessor 410 ausgeführt. Das Betriebssystem von Vorrichtung 400 ist beispielsweise ein Windows -, UNIX-, Linux-, Android-, Apple iOS- und/oder MAC-Betriebssystem.

Das Betriebssystem ermöglicht insbesondere die Verwendung der Vorrichtung 400 zur Datenverarbeitung. Es verwaltet beispielsweise Betriebsmittel wie Hauptspeicher 411 und Programmspeicher 412, Netzwerkschnittstelle 413, Ein- und Ausgabegerät 414, stellt unter anderem durch Programmierschnittstellen anderen Programmen grundlegende Funktionen zur Verfügung und steuert die Ausführung von Programmen.

Prozessor 410 steuert die Kommunikationsschnittstelle 413, welche beispielsweise eine Netzwerkschnittstelle sein kann und als Netzwerkkarte, Netzwerkmodul und/oder Modem ausgebildet sein kann. Die Kommunikationsschnittstelle 413 ist insbesondere dazu eingerichtet, eine Verbindung der Vorrichtung 400 mit anderen Vorrichtungen, insbesondere über ein (drahtloses) Kommunikationssystem, beispielsweise ein Netzwerk, herzustellen und mit diesen zu kommunizieren. Die Kommunikationsschnittstelle 413 kann beispielsweise Daten (über das Kommunikationssystem) empfangen und an Prozessor 410 weiterleiten und/oder Daten von Prozessor 410 empfangen und (über das Kommunikationssystem) senden. Beispiele für ein Kommunikationssystem sind ein lokales Netzwerk (LAN), ein großräumiges Netzwerk (WAN), ein drahtloses Netzwerk (beispielsweise gemäß dem IEEE-802.11-Standard, dem Bluetooth (LE)-Standard und/oder dem NFC-Standard), ein drahtgebundenes Netzwerk, ein Mobilfunknetzwerk, ein Telefonnetzwerk und/oder das Internet.

Des Weiteren kann Prozessor 410 zumindest ein Ein-/Ausgabegerät 414 steuern. Ein-/Ausgabegerät 414 ist beispielsweise eine Tastatur, eine Maus, eine Anzeigeeinheit, ein Mikrofon, eine berührungsempfindliche Anzeigeeinheit, ein Lautsprecher, ein Lesegerät, ein Laufwerk und/oder eine Kamera bzw. Ausgabegeräte der Vorrichtung für Körperübungen. Ein-/Ausgabegerät 414 kann beispielsweise Eingaben eines Benutzers aufnehmen und an Prozessor 410 weiterleiten und/oder Informationen für den Benutzer von Prozessor 410 empfangen und ausgeben, beispielsweise als Sensormittel der Vorrichtung für Körperübungen.

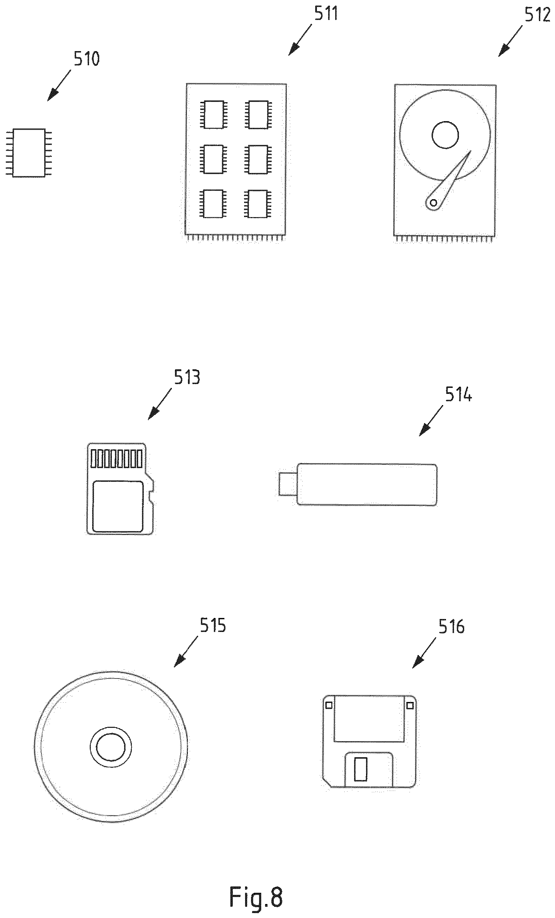

Fig. 8 zeigt schließlich unterschiedliche Ausführungsbeispiele von Speichermedien, auf denen ein Ausführungsbeispiel eines Computerprogrammes gespeichert sein kann. Das Speichermedium kann beispielsweise ein magnetisches, elektrisches, optisches und/oder andersartiges Speichermedium sein. Das Speichermedium kann beispielsweise Teil eines Prozessors (z.B. des Prozessor 410 der Fig. 7) sein, beispielsweise ein (nicht-flüchtiger oder flüchtiger) Programmspeicher des Prozessors oder ein Teil davon (wie Programmspeicher 412 in Fig. 7). Ausführungsbeispiele eines Speichermediums sind ein Flash-Speicher 510, eine SSD-Festplatte 511, eine magnetische Festplatte 512, eine Speicherkarte 513, ein Memory Stick 514 (z.B. ein USB-Stick), eine CD-ROM oder DVD 515 oder eine Diskette 516.

**Patentansprüche**

1. Vorrichtung für Körperübungen,

   - mit einem kugelförmigen Gehäuse (4),
   - mit Sensormitteln zum Bestimmen von Sensordaten indikativ für eine Einwirkung eines Benutzers auf die Vorrichtung,
   - mit Ausgabemitteln zur Ausgabe von Ausgabeinformation an den Benutzer und
   - mit einer Steuereinrichtung (30),
   - wobei die Steuereinrichtung (30) dafür eingerichtet ist, die Ausgabemittel in Abhängigkeit der Sensordaten zu steuern,

   **dadurch gekennzeichnet, dass**

   - die Sensormittel mindestens einen Näherungssensor umfassen, welcher dafür eingerichtet ist, Sensordaten indikativ für eine Entfernung der Einwirkung des Benutzers zur Vorrichtung bereitzustellen, wobei über den mindestens einen Näherungssensor eine Entfernung der Einwirkung des Benutzers zur Vorrichtung von bis zu 10 cm detektiert wird, wobei innerhalb dieser Entfernung eine quantitative Entfernungsinformation bereitgestellt wird,
   - dass der mindestens eine Näherungssensor als kapazitiver Näherungssensor ausgestaltet

ist,
- dass der mindestens eine Näherungssensor als Kugelabschnitt an der Innenfläche der Außenwand des Gehäuses (4) angeordnet ist, und eine Bestimmung einer Richtungsabhängigkeit der Einwirkung des Benutzers erlaubt, und
- der kapazitative Näherungssensor mehrere Kondensatorbereiche (28a, 28b) mit jeweils mindestens zwei Kondensatorelektroden (14a, 14c; 14b, 14d) aufweist.

2. Vorrichtung nach Anspruch 1,
**dadurch gekennzeichnet, dass**
der kapazitative Näherungssensor über einen Schwingkreis (46) angeregt wird, insbesondere unter Verwendung mindestens eines Schmitt-Triggers (IC1, 1C2).

3. Vorrichtung nach einem der Ansprüche 1 bis 2,
**dadurch gekennzeichnet, dass**
der Näherungssensor dafür eingerichtet ist, Sensordaten indikativ für eine Richtungsabhängigkeit der Einwirkung des Benutzers bereitzustellen.

4. Vorrichtung nach einem der Ansprüche 1 bis 3,
**dadurch gekennzeichnet, dass**
die Sensormittel weiter mindestens einen Drucksensor (26, 36), mindestens ein Beschleunigungssensor, mindestens ein Gyroskop, mindestens einen Kompass (40), mindestens einen Geschwindigkeitssensor, mindestens ein Positionsbestimmungsmittel und/oder mindestens ein akustisches Sensormittel (38) umfassen.

5. Vorrichtung nach einem der Ansprüche 1 bis 4,
**dadurch gekennzeichnet, dass**
das Gehäuse (4) austauschbar ausgestaltet ist.

6. Vorrichtung nach einem der Ansprüche 1 bis 5,
**dadurch gekennzeichnet, dass**
die Ausgabemittel zur Ausgabe von optischer, mechanischer und/oder akustischer Ausgabeinformation eingerichtet sind.

**Claims**

1. Device for physical exercises,

- with a spherical housing (4),
- with sensor means for determining sensor data indicative of a user's effect on the device,
- with output means for outputting output information to the user and
- with a control device (30),
- wherein the control device (30) is configured to control the output means as a function of the sensor data,

**characterized in that**

- the sensor means comprise at least one proximity sensor which is configured to provide sensor data indicative of a distance of the user's effect to the device, wherein a distance of the user's effect to the device of up to 10 cm is detected via the at least one proximity sensor, wherein quantitative distance information is provided within this distance,
- **in that** the at least one proximity sensor is designed as a capacitive proximity sensor,
- **in that** the at least one proximity sensor is arranged as a spherical section on the inner surface of the outer wall of the housing (4), and permits a determination of a directional dependency of the user's effect, and
- the capacitive proximity sensor has several capacitor regions (28a, 28b), each with at least two capacitor electrodes (14a, 14c; 14b, 14d).

2. Device according to claim 1,
**characterized in that**
the capacitive proximity sensor is excited via an oscillating circuit (46), in particular using at least one Schmitt trigger (IC1, IC2).

3. Device according to one of claims 1 to 2,
**characterized in that**
the proximity sensor is configured to provide sensor data indicative of a directional dependency of the user's effect.

4. Device according to one of claims 1 to 3,
**characterized in that**
the sensor means further comprise at least one pressure sensor (26, 36), at least one acceleration sensor, at least one gyroscope, at least one compass (40), at least one speed sensor, at least one position determining means and/or at least one acoustic sensor means (38).

5. Device according to one of claims 1 to 4,
**characterized in that**
the housing (4) is designed to be replaceable.

6. Device according to one of claims 1 to 5,
**characterized in that**
the output means are configured to output optical, mechanical and/or acoustic output information.

**Revendications**

1. Dispositif pour des exercices physiques,

- avec un boîtier sphérique (4),
- avec des moyens de détection pour déterminer des données de détection indicatives d'une effet d'un utilisateur sur le dispositif,
- avec des moyens de sortie pour la sortie des informations de sortie à l'utilisateur et
- avec un dispositif de commande (30),
- le dispositif de commande (30) étant configuré pour commander les moyens de sortie en fonction des données de détection,

**caractérisé en ce que**

- les moyens de détection comprennent au moins un capteur de proximité qui est configuré pour fournir des données de détection indicatives d'une distance de l'effet de l'utilisateur par rapport au dispositif, une distance de l'effet de l'utilisateur par rapport au dispositif allant jusqu'à 10 cm étant détectée par l'au moins un capteur de proximité, une information de distance quantitative étant fournie dans les limites de cette distance,
- **en ce que** l'au moins un capteur de proximité est conçu comme un capteur de proximité capacitif,
- **en ce que** l'au moins un capteur de proximité est disposé sous la forme d'une section sphérique sur la surface intérieure de la paroi extérieure du boîtier (4), et permet de déterminer une dépendance directionnelle de l'effet de l'utilisateur, et
- le capteur de proximité capacitif présente plusieurs zones de condensateur (28a, 28b) avec respectivement au moins deux électrodes de condensateur (14a, 14c; 14b, 14d).

2. Dispositif selon la revendication 1, **caractérisé en ce que** le capteur de proximité capacitif est excité par un circuit oscillant (46), en particulier en utilisant au moins un bascule de Schmitt (IC1, IC2).

3. Dispositif selon l'une des revendications 1 à 2, **caractérisé en ce que** le capteur de proximité est configuré pour fournir des données de détection indicatives d'une dépendance directionnelle de l'effet de l'utilisateur.

4. Dispositif selon l'une des revendications 1 à 3, **caractérisé en ce que** les moyens de détection comprennent en outre au moins un capteur de pression (26, 36), au moins un capteur d'accélération, au moins un gyroscope, au moins une boussole (40), au moins un capteur de vitesse, au moins un moyen de détermination de position et/ou au moins un moyen de détection acoustique (38).

5. Dispositif selon l'une des revendications 1 à 4, **caractérisé en ce que** le boîtier (4) est conçu de manière interchangeable.

6. Dispositif selon l'une des revendications 1 à 5, **caractérisé en ce que** les moyens de sortie sont configurés pour la sortie des informations de sortie optiques, mécaniques et/ou acoustiques.

Fig.1

Fig.2

Fig.3

Fig.4

Fig.5

48

52
Hinterlegen von Nutzerprofilen

50 → Erhalten eines Nutzerprofils

54 — Auswahl einer Ausgabe-funktion aus vorbestimmten Vorgabefunktionen

56

58 — Bestimmen von Sensordaten

66
Ermitteln einer Historie von Sensordaten

60 — Erhalten von Sensordaten

62 — Bestimmen von Ausgabe-information

64 — Ausgabe der Ausgabe-information

Fig.6

Fig.7

Fig.8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0108755 A1
- WO 2017152917 A1
- US 20170188864 A1
- US 20130057503 A1
- US 5512836 A